# EUROPÄISCHE PATENTSCHRIFT

(11) **EP 0 524 971 B1**
(45) Veröffentlichungstag und Bekanntmachung des Hinweises auf die Patenterteilung: **22.01.1997**
(21) Anmeldenummer: 91906928.6
(22) Anmeldetag: 13.04.1991
(51) Int. Cl.: A61L 2/00, A61L 2/18

(54) **DEKONTAMINIERTES BIOLOGISCHES MATERIAL**
DECONTAMINATED BIOLOGICAL MATERIAL
MATERIAU BIOLOGIQUE DECONTAMINE

(30) Priorität: 18.04.1990 DE 4012323; 09.05.1990 DE 4014063
(43) Veröffentlichungstag der Anmeldung: 03.02.1993
(73) Patentinhaber: BOHN, Burghard, D-69126 Heidelberg (DE)
(72) Erfinder: BOHN, Burghard, D-69126 Heidelberg (DE)
(74) Vertreter: Schmid, Rudolf, Dipl.-Ing., Patentanwalt
(86) Internationale Anmeldenummer: DE9100307
(87) Internationale Veröffentlichungsnummer: WO9116082

(56) Entgegenhaltungen:
- GB-A- 1 471 336
- US-A- 3 227 626
- US-A- 4 841 023

## Beschreibung

In zahlreichen Bereichen von Forschung und Entwicklung sowie industriellen und gewerblichen Bereichen wird Serum, insbesondere Kälberserum und fötales Kälberserum eingesetzt.
Dieses Serum wird in Zellkulturen zur Züchtung von menschlichen und tierischen Zellen verwendet. Es kommt weiterhin zum Einsatz bei der Herstellung von Proteinen und ähnlichen Pharmaprodukten, die mittels Zellkulturen fusionierter Zellen menschlichen oder tierischen, gegebenenfalls auch Insekten-Ursprungs, hergestellt werden.

Die Qualität derartigen Serums hängt von seinen Inhaltsstoffen ab; von besonderer Bedeutung dabei sind beispielsweise Insulin und Transferrin.

Serum, insbesondere Kälberserum und fötales Kälberserum sind insbesondere hinsichtlich wichtiger biologischer Komponenten, hitzeempfindlich.

Demzufolge ist es beispielweise nicht möglich, durch Temperaturerhöhung bzw. durch Erhitzung des Serums und dergleichen eine Dekontamination dieser biologischen Materialien zu erreichen.

Eine solche Dekontamination ist erforderlich, um insbesondere Viren, wie beispielsweise Maul- und Klauenseuche-Virus, Retroviren, beispielsweise HIV-Virus oder verwandte Viren, sowie sonstige infektiöse Agenzien zu inaktivieren.

Zur Inaktivierung genügt es beispielsweise nicht, eine sogenannte "Pasteurisierung" bei 60° C durchzuführen. Auch ist es nicht möglich, durch Erhöhen über diese Pasteurisierungstemperatur, z. B. durch Erhitzen auf 65° C für 2 mal eine halbe Stunde eine biologische Dekontamination in zufriedenstellender Weise durchzuführen. Es werden durch diese denaturierenden Bedingungen biologische Aktivitätsänderungen bewirkt, die beispielsweise auf einer Depolymerisierung von Aggregaten und der Denaturierung von Proteinen beruhen.

Zu den Verfahren des Standes der Technik ist insbesondere zu erwähnen die biologische Dekontamination, insbesondere die Inaktivierung von Viren, mittels Röntgenstrahlung. Dazu ist zu nennen beispielsweise die Literaturstelle "Canadian Journal of comparative Medicine, 45: 397-399 (1981).

Die Bestrahlung biologischen Materials mit den gegebenenfalls erforderlichen hohen Dosen zur Virenzerstörung ist der Qualität des biologischen Materials abträglich.

Auch die zur biologischen Dekontamination, insbesondere zur Inaktivierung von Viren eingesetzten chemischen Agenzien, wie beispielsweise Ethylenoxid bewirken einerseits eine in manchen Fällen unzureichende Dekontamination und andererseits eine Beeinträchtigung des biologischen Materials Unter der Beeinträchtigung des biologischen Materials ist aufgrund der geschilderten Änderung der Komponenten beispielsweise eine Beeinträchtigung der Förderung von Zellwachstum zu verstehen.

Die Verfahren des Standes der Technik zur biologischen Dekontamination biologischen Materials, insbesondere biologischer Flüssigkeit, sind somit mit den Nachteilen unzureichender Wirksamkeit und / oder Beeinträchtigungen der Qualität des biologischen Materials bzw. seiner Komponenten behaftet.

Zum Stand der Technik sind ferner die GB-A-1 471 336 und die US-A-3 227 626 zu nennen.

Die GB-A-1 471 336 beschreibt ein Verfahren zur Sterilisierung von säureres istentem, biologischem Material, bei dem das biologische Material auf einen pH-Wert von 1,25 bis 2,5 angesäuert wird, mit anschließendem Aufbewahren des biologischen Materials, bei einer Temperatur von 4 bis 70 °C, innerhalb einer Zeit von bis zu 48 Stunden, mit dem Zweck, die in ihm enthaltene mikrobiologische Flora, nicht jedoch das biologische Material zu inaktivieren; sodann erfolgt die Neutralisierung des biologischen Materials.

Diese Behandlungsweise ist jedoch für Seren nicht geeignet, da man bei diesen nicht bis zu einem pH-Wert von 1,5 heruntergehen darf. Es würde nämlich bei einem pH-Wert von 1,5 nachteiligerweise eine Verklumpung bzw. eine teilweise irreversible Denaturierung des biologischen Materials stattfinden, womit das Erfindungsziel nicht mehr erreichbar wäre.

Auch wird die erfindungsgemäß vorzunehmende Bestimmung der Rest-Aktivität von LDH (Lactat-Dehydrogenase) im Dokument GB-A-1 471 336 nicht durchgeführt.

Ein weiterer fundamentaler und patentbegründender Effekt besteht darin, daß die in Seren als störend oder qualitätsmindernd empfundenen bakteriellen Endotoxine,durch die erfindungsgemäße Behandlungsmethode in ihrer Konzentration sprunghaft vermindert werden.

Das Dokument US-A-3 227 626 beschreibt ein Verfahren zur Sterilisierung von Plasminogen durch 10-stündiges Erhitzen einer wässrigen Zubereitung auf 60 °C, wobei die Zubereitung eine molare Lysin-Konzentration von 0,1 bis 0,5, einen Plasminogengehalt von 0,25 bis 20 mg pro ml und einen pH-Wert von 5,3 bis 7,5 aufweist.
Auch dieses Dokument US-A-3 227 626 beschreibt weder die erfindungsgemäße Behandlung mit Säure, gefolgt von einer Laugenbehandlung, noch eine Bestimmung der Restaktivität der LDH (Lactat-Dehydrogenase).

Demgegenüber liegt vorliegender Erfindung die Aufgabe zugrunde, ein Verfahren zur biologischen Dekontamination zu liefern und dekontaminiertes biologisches Material zur Verfügung zu stellen, wobei in effektiver und schonender Weise die biologische Dekontamination, insbesondere die Viren-Inaktivierung, erzielt wird.

Demzufolge betrifft vorliegende Erfindung dekontaminierte Seren, die dadurch gekennzeichnet sind, daß sie durch Behandlung mit Säure, gefolgt von Laugenbehandlung, nämlich durch Behandlung mit einer Säure niedriger Normalität zum Absenken des pH-Werts auf 4,5 bis 5,5 für 2,5 bis 4 Stunden und anschließender Anhebung des pH-Werts durch Laugenbehandlung auf 7,0 bis 7,5 erhältlich sind, wobei der Behandlungsabschluß durch Bestimmung der Restaktivität der LDH (Lactat-Dehydrogenase) bestimmt wird.

Dabei wird nach einer bevorzugten Ausführungsform die LDH-Aktivität durch die Säure-/Laugenbehandlung um den Faktor 4 bis 15, vorzugsweise 8 bis 12 verringert.

Nach einer weiteren Ausführungsform wird die Abnahme der LDH-Aktivität durch Aktivitätsmessung vor und nach der Säure-/Laugenbehandlung bestimmt.

Die Erfindung betrifft also ferner auch ein Verfahren zur Dekontamination von Seren, wobei der pH-Wert für 2,5 bis 4 Stunden auf 4,5 bis 5,5 abgesenkt und anschließend durch Laugenzugabe auf 7,0 bis 7,5 angehoben wird und der Behandlungs-Verlauf und/oder der Behandlungs-Abschluß durch Bestimmung der Restaktivität von LDH (Lactat-Dehydrogenase) verfolgt bzw. bestimmt wird.

Es zeigte sich, daß die Inaktivierung von Viren, beispielsweise von saurelabilen Retroviren, sprunghaft gesteigert wird.
Dabei werden, wie Zellkulturzüchtungsversuche gezeigt haben, die biologischen Komponenten des Serums nicht beeinträchtigt.
Somit besitzt ein erfindungsgemäßes bzw. erfindungsgemäß behandeltes Serum, insbesondere Kälberserum und fötales Kälberserum, eine sprunghaft gesteigerte Wirkung beim Einsatz in Forschung und Industrie.

Es ist insbesondere eine positive Eigenschaft der vorliegenden Erfindung, daß die Ionenstärke des erfindungsgemäßen bzw. erfindungsgemäß behandelten Serums sich nur geringfügig ändert und damit nur geringe Osmolaritätsänderungen auftreten, beispielsweise im Bereich von 20 bis 30 m Osmol.
Beim erfindungsgemäßen Verfahren bzw. Produkt wird die Pufferkapazität relativ rasch durchbrochen und eine Absenkung des pH's erzielt, wobei durch die erfindungsgemäßen Parameter die Inaktivierung beispielsweise von Viren und generell die biologische Dekontamination erzielt wird.

Die Erfindung betrifft eine pH-Wert-Absenkung mit Säure, insbesondere Mineralsäure niedrigerer Normalität, beispielsweise mit 1 N Salzsäure, Halten des somit behandelten Materials bei niedrigem pH für beispielsweise 3 Stunden, und pH-Wert-Anhebung.
Ein besonders günstiger pH-Wert ist der pH 4,9. Die Temperatur wird dabei gehalten in einem Bereich von Kühlschranktemperatur, circa + 4 °C, und der Raumtemperatur; demzufolge bleiben die thermolabilen biologischen Komponenten des Serums unversehrt; auch die bei abgesenktem pH-Wert thermolabilen biologischen Komponenten des Serums bleiben unversehrt.

Mit dem erfindungsgemäßen Verfahren bzw. beim erfindungsgemäßen Produkt sind alle Seuchengesetzrelevanten Viren sowie Retroviren wirkungsvoll inaktivierbar bzw. inaktiviert.

Zur Anhebung des pH's auf einen gewünschten, insbesondere physiologischen Wert von etwa 7 bis 7,5 wird eine Base niedriger Normalität, insbesondere 1 N Natronlauge verwendet.

Zur Absenkung des pH's können auch sonstige geeignete Säuren entsprechender Dissoziationskonstante eingesetzt werden.
Auch können sonstige Basen wie beispielsweise Kalilauge oder organische Basen eigesetzt werden.

Die Wahl der geeigneten Base bzw. der geeigneten Säuren hängt gegebenenfalls von der Zweckbestimmung des Serums hinsichtlich Zellkulturen, Medienzusammensetzung, Ionengehalt und dergleichen ab.
Eine geeignete Auswahl ist für den speziellen Fall dem Fachmann ohne weiteres aufgrund seines allgemeinen Fachwissens möglich.

Das erfindungsgemäße Verfahren ermöglicht es, auch Seren bzw. fötales Kälberserum einer Verwendung in Zellkulturen sowie im Industrie- und Pharmabereich und in der Forschung zuzuführen, das aus Regionen mit hoher potentieller biologischer Kontaminationbelastung kommt.

Im folgenden ist ein Ausführungsbeispiel der Erfindung dargelegt.

Ein Liter eines fötalen Kälberserums wird mit steriler ein normaler HCl auf einen pH von 4,9 abgesenkt und für zwei Stunden bei diesem Wert bei + 4 °C (Kühlraum) gehalten.

Anschließend wird der pH durch Zugabe von steriler 1 N NaOH auf einen Wert von pH 7,3 wieder angehoben.

Das somit erhaltene Material wird zur Züchtung von adhärenten Zellen verwendet.

Dabei werden in eine Zellkulturschale mit 20 ml Flüssigkeits-Kulturvolumen und 10 % Serumgehalt 1 x 10 Zellen eingesät.
Nach 2 Tagen wird eine Zellzahl von 3,2 x 10 , nach 5 Tagen von 2,3 x 10 und nach 7 Tagen von 4,8 x 10 erzielt.

Die Zellen zeigen ein gesundes Aussehen; cytopathische oder virus-spezifische Effekte sind nicht beobachtbar.

Durchgeführte Versuche bzw. Tests zur Anwesenheit und Abwesenheit bzw. Inaktivierung, wurden mit Maul- und - Klauenseuche-Virus und Retroviren durchgeführt und zeigen vollständige Inaktivierung bzw. Entfernung der Viren.

Die vorliegende Erfindung betrifft also ferner auch eine Möglichkeit zur Überprüfung des Behandlungs-Zustandes bzw. der Durchführung einer Behandlung unter Nutzung eines "Leitenzyms".
Die LDH (Lactat-Hydrogenase) ist ein Enzym, das in Kälberseren und Rinderseren ubiquitär vorhanden ist und beispielsweise im Kälberserum in einer Konzentration von etwa 100 U/l und in Rinderserum einer Konzentration von etwa 100 bis 500 U/l vorliegt.
Die LDH ist für die gewünschten Wirkungen von Seren, insbesondere bei der Zellzüchtung und für das Zellwachstum, ohne Bedeutung.
Demzufolge ist ihre Abnahme für die Qualität des Serums bzw. der Seren nicht relevant, im Gegenteil, da potentielle Nebenwirkungen einer enzymatischen Aktivität der LDH reduziert werden, stellt das erfindungsgemäße biologische Material bzw. das erfindungsgemäße Verfahren eine Verbesserung der Qualität der Seren bzw. der biologischen Materialien dar.

In der Praxis ist es empfehlenswert, entweder im Rahmen von LDH- Aktivitätsmessungen vor und nach einer Behandlung oder im Rahmen einer Messung der Seren und den dazugehörigen Rückstellmustern die Abnahme an LDH-Aktivität zu bestimmen.
Weiterhin bietet sich an, eine, für jeweilige biologische Materialien, spezifische Grenze bzw. einen Grenzwert, beispielsweise von kleiner gleich 40 U/l bei Kälberserum als Messgrenze bzw. als Grenzwert festzulegen.

Es folgen Ausführungsbeispiele der vorliegenden Erfindung:

### Beispiel 1

Fötales Kälberserum, das einer dreistündigen Behandlung, bei 4°C, und einem pH-Wert von 4,9 (gefolgt von anschließender pH-Anhebung) unterworfen wurde, wurde hinsichtlich der LDH-Aktivität vor und nach der Behandlung gemessen.
Die LDH-Aktivität betrug 100 U/l vor der Behandlung und 8,3 U/l nach der Behandlung.

Der Pyrogengehalt sank gleichzeitig von 1,2 ng/ml vor, auf 0,3 ng/ml nach der Behandlung.

### Beispiel 2

Ein Rinderserum wurde analog zu Beispiel 1 behandelt und vor bzw. nach Behandlung der LDH-Gehalt gemessen. Der Wert betrug 350 U/l vor und 25 U/l nach der Behandlung.

### Beispiel 3

Ein Rinderserum wurde gemäß Beispiel 1 behandelt. Der LDH-Wert vor Behandlung betrug 300 U/l und nach der Behandlung 16,6 U/l.

## Patentansprüche

1. Dekontaminierte Seren,
dadurch gekennzeichnet,
daß sie durch Behandlung mit Säure, gefolgt von Laugenbehandlung, nämlich durch Behandlung mit einer Säure niedriger Normalität zum Absenken des pH-Werts auf 4,5 bis 5,5 für 2,5 bis 4 Stunden und anschließender Anhebung des pH-Werts durch Laugenbehandlung auf 7,0 bis 7,5 erhältlich sind, wobei der Behandlungsabschluß durch Bestimmung der Restaktivität der LDH (Lactat-Dehydrogenase) bestimmt wird.

2. Dekontaminierte Seren nach Anspruch 1,
dadurch gekennzeichnet,
daß die LDH-Aktivität durch die Säure-/Laugenbehandlung um den Faktor 4 bis 15, vorzugsweise 8 bis 12, verringert wird.

3. Dekontaminerte Seren nach Anspruch 1 oder 2,
dadurch gekennzeichnet,
daß die Abnahme der LDH-Aktivität durch Aktivitätsmessung vor und nach der Säure-/Laugenbehandlung bestimmt wird.

4. Verfahren zur Dekontamination von Seren,
wobei der pH-Wert für 2,5 bis 4 Stunden auf 4,5 bis 5,5 abgesenkt und anschließend durch Laugenzugabe auf 7,0 bis 7,5 angehoben wird und der Behandlungs-Verlauf und/oder der Behandlungs-Abschluß durch Bestimmung der Restaktivität von LDH (Lactat-Dehydrogenase) verfolgt bzw. bestimmt wird.

5. Verfahren nach Anspruch 4,
dadurch gekennzeichnet,
daß die LDH-Aktivität durch die Säure-/Laugenbehandlung um den Faktor 4 bis 15, vorzugsweise 8 bis 12 verringert wird.

6. Verfahren nach Ansprüchen 4 oder 5,
dadurch gekennzeichnet,
daß die Abnahme der LDH-Aktivität durch Aktivitätsmessung vor und nach der Säure-/Laugenbehandlung bestimmt wird.

## Claims

1. Decontaminated sera,
**wherein**
they are obtainable by a treatment with acid, followed by a treatment with an alkaline liquor, namely an acid having a low normality used to lower the pH to a value between 4.5 and 5.5 during 2.5 to 4 hours, followed by a treatment with an alkaline liquor used to rise the pH to a value between 7.0 and 7.5, with the end of such treatment being defined by a determination of the LDH (lactate dehydrogenase) residual activity.

2. Decontaminated sera according claim 1,
**wherein**
the LDH activity is reduced, by the treatment with acid/alkaline liquor, by the factor 4 to 15, preferably by the factor 8 to 12.

3. Decontaminated sera according claims 1 or 2,
**wherein**
the decrease of the LDH activity is determined by measuring the activity before and after the treatment with acid/alkaline liquor.

4. Procedure for the decontamination of sera,
**wherein**
the pH is lowered to a value between 4.5 and 5.5 for a period of 2.5 to 4 hours and subsequently rised, by the addition of an alkaline liquor, to a value between 7.0 and 7.5, with the progress and/or the end of the treatment being followed up, or defined, by a determination of the LDH (lactate dehydrogenase) residual activity.

5. Procedure according to claim 4,
**wherein**
the LDH activity is reduced, by the treatment with acid/alkaline liquor, by the factor 4 to 15, preferably by the factor 8 to 12.

6. Procedure according to claims 4 or 5,
**wherein**
the decrease of the LDH activity is determined by measuring the activity before and after the treatment with acid/alkaline liquor.

## Revendications

1. Sérums décontaminés,
caractérisés en ce
qu'ils sont disponibles, après traitement à l'acide puis lavage alcalin, à savoir après traitement avec un acide de faible normalité pour réduire le pH à une valeur comprise entre 4,5 et 5,5 pendant une durée allant de 2,5 à 4 heures puis élévation du pH par alcalisation pour obtenir des valeurs variant entre 7,0 et 7,5, la fin du traitement étant déterminée par évaluation de l'activité résiduelle de la LDH (déhydrogénase de lactate).

2. Sérums décontaminés selon la revendication 1,
caractérisés en ce
que l'activité de la LDH est réduite d'un facteur compris entre 4 et 15, de préférence entre 8 et 12, par traitement à l'acide/lavage alcalin.

3. Sérums décontaminés selon la revendication 1 ou 2,
caractérisés en ce
que la baisse d'activité de la LDH est déterminée par mesure de l'activité avant et après le traitement à l'acide/lavage alcalin.

4. Procédé de décontamination de sérums
comprenant l'abaissement du pH à une valeur comprise entre 4,5 et 5,5 pendant 2,5 à 4 heures et l'ajout ultérieur de bases pour relever le pH à un niveau variant 7,0 et 7,5. Le déroulement du traitement et/ou la fin de celui-ci sont suivis et déterminés par mesure de l'activité résiduelle de la LDH (hydrogénase de lactate).

5. Procédé selon revendication 4,
caractériséen ce
que l'activité de la LDH est réduite d'un facteur allant de 4 à 15, de préférence de 8 à 12, par traitement à l'acide/lavage alcalin.

6. Procédé selon les revendications 4 ou 5,
caractérisé en ce
que la baisse de l'activité de la LDH est déterminée par la mesure de l'activité avant et après traitement à l'acide/lavage alcalin.
